# EUROPÄISCHE PATENTSCHRIFT

(11) **EP 1 524 273 B1**
(45) Veröffentlichungstag und Bekanntmachung des Hinweises auf die Patenterteilung: **22.11.2006**
(21) Anmeldenummer: 03090344.7
(22) Anmeldetag: 13.10.2003
(51) Int. Cl.: C07F 15/00, A61P 35/00

(54) **Verfahren zur Herstellung von trans- oder cis-Diammoniumdichlorodihydroxoplatin (IV)-Salzen und -Derivaten und ihre Verwendung zur Herstellung von pharmazeutischen Wirkstoffen**
Process for the preparation of trans- or cis-diammoniumdichlorodihydroxyplatinum(IV) salts and derivatives and their use for the preparation of pharmaceutical active agents
Procédure pour la preparation des sels et derivées de trans- et cis-diammoniumdichlorodihydroxyplatine et leur usage pour la preparation des agents pharmaceutique

(43) Veröffentlichungstag der Anmeldung: 20.04.2005
(73) Patentinhaber: Salama, Zoser B. nat.rer.Dr., 13465 Berlin (DE)
(72) Erfinder: Salama, Zoser B. nat.rer.Dr., 13465 Berlin (DE)
(74) Vertreter: Hertin, Paul W.

(56) Entgegenhaltungen:
- SU-A- 1 137 698
- R.L. KELLAND ET AL: "Ammin/mine Platinum(IV) dicarboxylates: A novel class of platinum complex exhibiting selective cytotoxicitiy to intrinsically cis-platin-resistent human ovarian carcinoma cell lines" CANCER RESEARCH, Bd. 52, Nr. 4, 1992, Seiten 822-828, XP001180227

## Beschreibung

Die Erfindung betrifft ein Verfahren zur Herstellung von trans- oder cis-Diammoniumdichlorodihydroxoplatin (IV), Salze und Derivate, die sich aus dieser Verbindung ableiten; die Erfindung betrifft weiterhin die Verwendung dieser Verbindungen in der Prophylaxe, Therapie, Verlaufskontrolle und in der Nachbehandlung von mit Zellwachstum, Zelldifferenzierung und/oder Zellteilung im Zusammenhang stehenden Krankheiten, insbesondere Tumoren.

Es ist bekannt, dass Metalle, wie zum Beispiel Molybdän, Vanadium, aber insbesondere auch Gold und Platin, in der Therapie von akuten wie auch chronischen Erkrankungen, wie zum Beispiel Rheuma, Krebs oder auch Autoimmunerkrankungen, eingesetzt werden können. Viele der verwendeten Metalle werden in Form von Komplex-Verbindungen mit dem zu therapierenden Organismus - in vielen Fällen über die orale Aufnahme - in Kontakt gebracht.

Die die Metalle umfassenden Komplex-Verbindungen sind in der erforderlichen pharmazeutischen Reinheit nur sehr schwer herzustellen. Zahlreiche beschriebene Herstellungsverfahren generieren entweder die Verbindung nicht in einer ausreichenden pharmazeutischen Reinheit oder führen zu zahlreichen Nebenprodukten, die im Organismus zu nicht erwünschten Nebenwirkungen führen. Insbesondere die Herstellung von pharmazeutisch interessanten cis- und trans Oxo-Platin-Verbindungen ist mit bekannten Verfahren in der erforderlichen Reinheit oder Abwesenheit von Nebenprodukten nicht möglich (SU 1137698 A1, SU 1021116 A1).

Im Stand der Technik sind verschiedene Acyl-Derivate von Cis-Platinverbindungen offenbart (Kelland et al., Cancer Research, 1992). Gemäß der Lehre von Kelland et al. sind nicht die Acylreste, sondern die Substituenten am Stickstoffrest für die Wirkung der Verbindungen auf Eierstockkrebs-Zelllinien wesentlich. Nach Ansicht der Autoren besteht ein enger Zusammenhang zwischen den Substituenten am Stickstoffrest und der zytotoxischen Aktivität der Verbindungen. Mit steigender Menge der Kohlenstoffatome, der Substituenten am Stickstoffrest steigt auch die zytotoxische Aktivität der Cis-Platinverbindungen. Das heißt, gemäß den Ausführungen von Kelland et al. wäre ein Fachmann nicht motiviert gewesen, Acylreste gegen andere Verbindungen, z.B. Alkylreste auszutauschen, da das Augenmerk des Fachmanns durch Kelland et al. auf die Substituenten am Stickstoffrest gerichtet gewesen ist. Ein Fachmann ist durch Kelland et al. dazu angehalten, Substituenten mit einer möglichst großen Anzahl von Kohlenstoffatomen als Substituent am Stickstoffrest in die jeweiligen Cis-Platinverbindungen einzubringen. Besonders vorteilhaft sind nach Kelland et al. Ringverbindungen; demgemäß ist ein Fachmann motiviert, weitere, insbesondere sehr große Ringverbindungen durch eine Amin-Ligand-Substitution in die Cis-Platinverbindungen einzubringen.

In der SU 1 137 698 A1 sind Herstellungsverfahren für Cis-Diaminodichlorodihydroxoplatin (IV) offenbart. Bei den offenbarten Herstellungsverfahren wird eine Reinheit von 99 bis 99,6% des gewünschten Endproduktes erreicht.

Aufgabe der Erfindung war es daher, ein Verfahren bereitzustellen, mit dem cis- oder trans-Oxo-Platin-Verbindungen einfach, sicher und effektiv bereitgestellt werden können.

Die Erfindung löst diese erfindungsgemäße Aufgabe durch ein Verfahren zur Herstellung von cis- oder trans-Diammoniumdichlorodihydroxoplatin (IV) bzw. -Diammoniumdichlorotransdihydrcxoplatin (IV) und deren Derivaten, wobei cis- oder trans-Diammoniumdichloroplatin (II) mit > 30%iger Peroxid umfassenden Lösung bei Temperaturen unter 30 °C umgesetzt, das hierdurch gewonnene Produkt mit einer Mineralsäure gelöst und anschließend mit einer alkalischen Lösung ausgefällt wird.

Überraschenderweise ist es durch die Kombination der Merkmale der > 30%igen Peroxid-umfassenden Lösung mit relativ niedrigen Temperaturen, das heißt Temperaturen unter 30 °C, möglich, Oxo-cis-Platin- und Oxo-trans-Platin-Verbindungen einfach und sicher so herzustellen, dass wenig unerwünschte Nebenprodukte anfallen. Bei dem erfindungsgemäßen Verfahren werden als Ausgangsverbindungen cis- oder trans-Diammoniumdichloroplatin (II)-Verbindungen eingesetzt, die in die entsprechende cis- oder trans-Form des Diammoniumdichlorotransdihydroxoplatin (IV) umgewandelt werden. Es ist vorteilhaft, wenn die Peroxid-umfassende Lösung über einen längeren Zeitraum, bevorzugt kontinuierlich, zu der Ausgangsverbindung zugegeben wird. Bei diesen Peroxid-umfassenden Lösungen kann es sich um Lösungen handeln, die während der Reaktion Peroxid freigeben oder sich aufgrund ihrer spezifischen Struktur funktionsanalog verhalten, wie zum Beispiel Wasserstoffperoxid-Lösungen oder Perchloressigsäure-Lösungen.

Die hohe Ausbeute und die Reinheit der Reaktionsprodukte ist bei dem erfindungsgemäßen Verfahren mit einer sehr sicher ablaufenden Reaktion kombiniert. Gegenüber den bekannten Verfahren wird beispielsweise Schaumbildung und das Verspritzen der Reaktionsmischung, welches ein Verlust von Platin zur Folge hat, verhindert. Die hohe Reinheit des gewonnenen Produktes ermöglicht eine gute Weiterverarbeitung zu pharmazeutischen Wirkstoffen wie zum Beispiel Salzen und Derivaten. Selbstverständlich ist das erfindungsgemäße Verfahren auch für die Herstellung der gewünschten Verbindungen im technischen oder großtechnischen Maßstab geeignet.

Bei dem erfindungsgemäßen Verfahren wird zu den Ausgangsstoffen bei niedrigen Temperaturen und gegebenenfalls unter Kühlung mehrmalig insbesondere hochkonzentrierte Wasserstoffperoxidlösung zugegeben und für einen längeren Zeitraum, beispielsweise mehrere Tage, umgesetzt. Durch diesen Reaktionsschritt kann ein solides Produkt - wie zum Beispiel ein erster Niederschlag - erhalten werden, welches durch Zugabe einer Mineralsäure gelöst wird. Anschließend erfolgt eine Ausfällung mit einer bevorzugt konzentrierten Alkalilösung.

Im Anschluss kann das so gewonnene Produkt gewaschen und im Vakuum getrocknet werden.

Das heißt, dass das durch die Umsetzung von cis- oder trans-Diammoniumdichloroplatin (II) gewonnene Produkt ist eine Lösung oder ein Feststoff, wobei das gewonnene Produkt bevorzugt ein erster Niederschlag ist. Das gewonnene Produkt wird mit einer Mineralsäure, beispielsweise Phosphorsäure oder Schwefelsäure, umgesetzt. Wenn es sich bei dem gewonnenen Produkt gemäß der Erfindung um einen Niederschlag handelt, so wird dieser Niederschlag durch die Mineralsäure weitestgehend gelöst. Der so mit einer Mineralsäure gelöste Niederschlag wird anschließend mit einer alkalischen Lösung, beispielsweise einer konzentrierten Natriumhydroxidlösung, zu einem zweiten Niederschlag ausgefällt. Die weitere Verarbeitung des ausgefällten cis- oder trans-Diammoniumdichlorodihydroxoplatin (IV) ergibt sich aus der gewünschten weiteren Verwendung des gewonnenen Reaktionsproduktes und ist dem Fachmann bekannt.

In einer bevorzugten Ausführungsform der Erfindung ist die Peroxid-umfassende Lösung eine hochkonzentrierte Wasserstoffperoxid-Lösung. Diese Wasserstoffperoxid-Lösung ist bevorzugt eine > 30%ige Wasserstoffperoxid-Lösung und besonders bevorzugt eine 35%ige Wasserstoffperoxid-Lösung. Dem Fachmann ist bekannt, dass die Wasserstoffperoxid-Lösung auch andere Komponenten umfassen kann, die ihre oxidierende Wirkung erhöhen. Es kann beispielsweise vorteilhaft sein, eine Lösung aus 30%igem Wasserstoffperoxid und 70%iger Perchloressigsäure zu verwenden. Selbstverständlich ist es auch möglich, eine Lösung zu verwenden, die Perchloressigsäure, aber keine Wasserstoffperoxid-Lösung umfasst. Dem Fachmann sind zahlreiche funktionsanaloge Peroxid generierende bzw. Peroxid bereitstellende Verbindungen und Lösungen neben Wasserstoffperoxid und Perchloressigsäure - wie zum Beispiel Natrium- oder Kaliumperoxid - bekannt.

In einer weiteren bevorzugten Ausführungsform der Erfindung erfolgt die Umsetzung des Diammoniumdichloroplatin (II) entweder in der cis- oder in der trans-Form mit der Peroxid-umfassenden Lösung, bevorzugt der Wasserstoffperoxid-Lösung, bei Raumtemperatur. Die Raumtemperatur im Sinne der Erfindung liegt zwischen 20 °C und 29 °C, bevorzugt zwischen 23 °C und 27 °C und ganz besonders bevorzugt ungefähr im Bereich von 24 °C bis 25 °C. Die Raumtemperatur, wie sie hier verstanden wird, ist jedoch kein starrer Temperaturbereich. Entsprechend anderer Verfahrensparameter kann die Raumtemperatur im Sinne der Erfindung auch niedriger bzw. höher sein.

Besonders bevorzugt ist es, die Umsetzung von cis- oder trans-Diammoniumdichloroplatin (II) bzw. das gesamte erfindungsgemäße Verfahren bei weniger als 20 °C, bevorzugt weniger als 15 °C, ganz besonders bevorzugt bei weniger als 10 °C und insbesondere bei weniger als 7 °C durchzuführen. Weiterhin kann es ganz besonders bevorzugt sein, die Reaktion bei Kühlung mit Eiswasser durchzuführen, so dass eine Temperatur von ungefähr 4 °C erreicht wird. Bei einer Kühlung, die jedoch nicht zwingend auf 4 °C erfolgen muss, wird die Entstehung zahlreicher Nebenprodukte vorteilhafterweise unterdrückt.

In einer weiteren bevorzugten Ausführungsform der Erfindung wird als Mineralsäure eine Säure eingesetzt, die in der Lage ist, das gewonnene Produkt aus der Reaktion von Peroxid-umfassender Lösung mit dem cis- bzw. trans-Diammoniumdichloroplatin (II) zu lösen. Hierbei handelt es sich bevorzugt um Schwefelsäure. Selbstverständlich können aber auch andere dem Fachmann bekannte Mineralsäuren, wie zum Beispiel Phosphorsäure, Salpetersäure, Kohlensäure oder andere eingesetzt werden. Die erforderliche Konzentration dieser Mineralsäuren kann durch den Fachmann leicht ermittelt werden. Die Mengen und die Konzentrationen dieser Säuren sollten so gewählt werden, dass insbesondere das gewonnene Produkt, vor allem wenn es sich um einen ersten Niederschlag handelt, gelöst werden kann. Vorteilhafterweise ist die Konzentration so zu wählen, dass nur sehr wenig von den Mineralsäuren eingesetzt werden muss; wie zum Beispiel eine gerade ausreichende Menge einer 0,5N Schwefelsäure.

In einer bevorzugten Ausführungsform der Erfindung wird als alkalische Lösung eine Natriumhydroxid-Lösung verwendet. Vorteilhafterweise ist diese Natriumhydroxid-Lösung eine 25%ige, 30%ige, 33%ige bzw. 35%ige Natriumhydroxid-Lösung. Dem Fachmann ist bekannt, dass er neben der Natriumhydroxid-Lösung auch alle anderen basischen Verbindungen einsetzen kann, um das mit der Mineralsäure gewonnene Produkt auszufällen. Hierbei ist die alkalische Lösung so zu wählen, dass das Endprodukt möglichst rein ist und dass möglichst wenig Nebenprodukte entstehen. Dem Fachmann ist es möglich, dies durch Routineversuche festzustellen.

Besonders bevorzugt ist es in einer weiteren Ausführungsform der Erfindung, trans- oder cis-Diammoniumdichloroplatin (II) unter mehrmaliger Gabe der Peroxid-umfassenden Lösung, insbesondere der hochkonzentrierten Wasserstoffperoxid-Lösung, über eine Dauer von 12 bis 96, bevorzugt von 24 bis 48 Stunden, unter Kühlung umzusetzen. Hierbei bedeutet Kühlung zunächst, dass die Temperatur 30 °C nicht dauerhaft überschreitet. Eine kurzzeitige Überschreitung dieser Temperatur kann jedoch unter Umständen möglich und gewünscht sein. Bevorzugte Kühlungen sind die Kühlungen, die sich im technischen Maßstab effektiv und sicher sowie kostengünstig realisieren lassen, wie zum Beispiel die Kühlung mit Eiswasser bzw. Eiswassersalzgemischen. Die Kühlung ist bevorzugt so gewählt, dass es möglich ist, insbesondere die hochkonzentrierte Wasserstoffperoxid-Lösung so mit den Ausgangsstoffen umzusetzen, dass eine gut steuerbare chemische Reaktion abläuft. Steuerbar heißt im Sinne der Erfindung, dass es zu keinen heftigen chemischen Umsetzungen dergestalt kommt, dass beispielsweise Platinmetalle in Form von Spritzern oder anders das Reaktionsgemisch verlassen. Je nach Konzentration und Wahl der Ausgangsstoffe kann es sich bei der bevorzugten gekühlten Temperatur um Temperaturen von weniger als 10 °C handeln, besonders bevorzugt um Temperaturen im Bereich von 3 °C bis 7 °C. Der Fachmann kann durch Analyse der Reinheit des Endproduktes und gegebenenfalls auftretender Nebenprodukte leicht feststellen, ob er die gesamte Reaktion oder nur einzelne Teilschritte der Reaktion bei diesen Temperaturen durchführt. So kann es zum Beispiel vorteilhaft sein, nur die Umsetzung der Ausgangsstoffe mit der Peroxid-umfassenden Lösung, wie zum Beispiel Wasserstoffperoxid-Lösung, bei Temperaturen im Bereich von 3 °C bis 7 °C durchzuführen; genauso vorteilhaft kann es jedoch auch sein, das gesamte erfindungsgemäße Verfahren in diesem Temperaturbereich durchzuführen.

Vorteilhafterweise ist in einer weiteren bevorzugten Ausführungsform der Erfindung durch das routinemäßige Finden einer geeigneten Temperatur von weniger als 30 °C und einer entsprechenden Konzentration der Peroxid-umfassenden Lösung eine Ausbeute der Reaktionsprodukte von mehr als 60 %, bevorzugt mehr als 65 % und ganz besonders bevorzugt von mehr als 67 % der theoretisch berechneten Menge an trans- oder cis-Diammoniumdichlorodihydroxoplatin (IV) erzielbar.

Die Erfindung betrifft auch Verbindungen der allgemeinen Formel 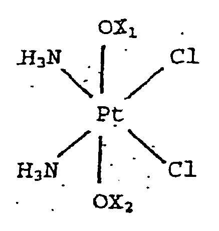 wobei x₁; x₂ ein Kation der 1. bis 5. Hauptgruppe oder der 1. bis 8. Nebengruppe des Periodensystems der Elemente, Alkyl und/oder Arylreste sein können, wobei bevorzugt x₁; x₂ = Calcium-, Magnesium-, Natrium-, Kalium-, Lithium-, Alkyl- und/oder Arylreste sein können.

Die Verbindungen dieser allgemeinen Formel können jeweils eine völlig unterschiedliche Struktur aufweisen. So ist es beispielsweise möglich, dass wenn x₁ und x₂ = Calcium- und/oder Magnesiumreste sind, dass jeweilige zweiwertige Kationen über Sauerstoffmoleküle zwei Platinkomplexe miteinander verbinden, so dass zwei Komplexverbindungen assoziiert vorliegen. Es kann aber auch möglichsein, dass, wenn x₁ und x₂ beispielsweise Natrium- oder Lithiumreste sind, eine Verbindung mit nur einem Platinkomplex vorliegt. Es kann bevorzugt auch möglich sein, dass x₂ ein zweiwertiges Kation, wie beispielsweise Calcium oder Magnesium ist, welches zwei Komplexe mit jeweils einem Zentralatom Platin miteinander verbindet und x₁ ein Natrium-, Kalium, Lithium-, Alkyl- und/oder Arylrest. Demgemäß ist es also möglich, dass aus der mit dem erfindungsgemäßen Verfahren gewonnenen Base trans- oder cis-Diammoniumdichlorodihydroxoplatin (IV) Salze mit ein-, zwei- oder dreiwertigen Ionen bzw. mit ein- oder zweiwertigen Ionen bzw. mit ein- und zweiwertigen Ionen und Alkyl- und/oder Arylresten in jeweils einer Verbindung gewonnen werden. Bevorzugte Anionen können neben den Chloriden auch Sulfate, Phosphate, Nitrate oder Carbonate sein. Bevorzugte Alkyl-Reste sind zum Beispiel Methyl-, Ethyl- oder Prophyl-Reste, bevorzugte Aryl-Reste sind Phenyl-, Naphthyl- oder Anthryl-Reste. Die Salze und Derivate, insbesondere wenn sie aus den Basen generiert werden, die mit dem erfindungsgemäßen Verfahren hergestellt wurden, weisen mehrere Vorteile gegenüber cis- oder trans-Diammoniumdichlorodihydroxoplatin (IV) auf. Überraschenderweise sind die Salze gegenüber der Base so gut löslich, dass sie vorteilhafterweise mit anderen aktiven Wirkstoffen oder Vitaminen bzw. anderen bekannten pharmazeutischen Mitteln, wie zum Beispiel Antitumormitteln, kombiniert werden können. Insbesondere sind die erfindungsgemäßen Salze überraschenderweise in 0,1 N HCl um ein Vielfaches besser löslich als die entsprechende Base. Eine derartige Konzentration entspricht weitestgehend der Konzentration der Magensäure, das heißt, die erfindungsgemäßen Salze werden im Magen sofort gelöst, insbesondere wenn sie als pharmazeutisches Mittel über die orale Aufnahme oder anders dorthin gelangen. Dies ist insbesondere vorteilhaft, um Tumoren des Magens zu behandeln oder um zu gewährleisten, dass die erfindungsgemäßen Verbindungen über die Magenresorption in verschiedene Bereiche des Organismus gelangen. Die Salze und Derivate haben eine höhere Bioverfügbarkeit und sind in geringeren Dosen höher wirksam als die Base, aus der sie gewonnen werden. Dadurch haben sie weniger Nebenwirkungen. Die Verweildauer in wichtigen Stoffwechselorganen - wie Leber oder Niere - der Salze ist auch geringer als die der Ursprungsverbindungen. Die Salze, insbesondere die Strukturen, in denen einwertige und zweiwertige Kationen zusammen eine erfindungsgemäße Verbindung bilden, erzeugen weniger Addukte, beispielsweise in der Niere und in der Leber, und sind daher weniger nephrotoxisch oder auch weniger toxisch für die Leber. Weiterhin sind sie besser für die Magen-Darm-Passage geeignet. Diese Vorteile sind jedoch nicht auf die Salze, die mit ein- und zweiwertigen Kationen gewonnen werden, beschränkt. Die erfindungsgemäßen Verbindungen können insbesondere mit Vorteil in der Tumorprophylaxe und -Therapie eingesetzt werden. Die Erfindung betrifft daher auch die Verwendung der erfindungsgemäßen Verbindungen in der Medizin, insbesondere zur Herstellung eines Arzneimittels.

Die erfindungsgemäßen Verbindungen werden in einer therapeutischen Menge mit einem Organismus in Kontakt gebracht; in solch einem Falle werden die erfindungsgemäßen Verbindungen als pharmazeutisches Mittel oder als Arzneimittel eingesetzt; demgemäß können diese Begriffe im Kontext der Erfindung synonym verwendet werden. Der Ausdruck therapeutische Menge bezeichnet wie hierin verwendet eine Menge, die Symptome einer Störung oder responsiven, pathologisch physiologischen Kondition verhindert oder verbessert. In bestimmten Ausführungsformen der vorliegenden Erfindung ist die verabreichte Menge ausreichend, einen Tumor im Wachstum zu hemmen, die im Wesentlichen die Ausbreitung eines Tumors, die Tumor-Angiogenese, die Tumor-Invasion und/oder die Tumor-Metastasierung im Rezipienten verhindert oder hemmt, wobei die Tumorerkrankungen ausgewählt sind aus der Gruppe der neoplastischen Tumoren, der endzündlichen Tumoren und/oder der Abzesse, Ergüsse und Ödeme. Die Erfindung betrifft demgemäß - wie bereits ausgeführt - pharmazeutische Mittel oder Arzneimittel, die die erfindungsgemäßen Verbindungen umfassen, gegebenenfalls zusammen mit pharmazeutischen Hilfsstoffen.

Die an einem Gesunden im Falle der Prophylaxe bzw. an einem Patienten im Falle der Therapie zu verwendende Menge an erfindungsgemäßen Verbindungen wird formuliert und die Dosis gemäß üblicher medizinischer Praxis festgesetzt, wobei die zu behandelnde Störung, der Zustand des einzelnen Patienten, die Verabreichungsstelle, das Verabreichungsverfahren und andere, den behandelnden Ärzten bekannte Faktoren berücksichtigt werden. In ähnlicher Weise hängt die Dosis der verabreichten erfindungsgemäßen Verbindungen von den Eigenschaften des Tumors ab, von der in vivo Halbwertszeit erfindungsgemäßen Verbindungen im Plasma wie auch von der Konzentration der erfindungsgemäßen Verbindungen in der Formulierung, dem Verabreichungsweg, der Stelle und der Rate der Dosierung, der klinischen Toleranz des jeweiligen Individuums (Mensch und Tier), der pathologischen Affektion des Patienten und dergleichen, wie es Ärzten bzw. anderen Fachleuten bekannt ist. Im Allgemeinen werden Dosierungen von etwa 0,1 bis 1000 mg pro Individuum und Verabreichung bevorzugt; besonders bevorzugt ist eine Dosierung von 10 bis 500 mg, ganz besonders bevorzugt von 200 bis 400 mg, insbesondere von 300 mg. Es können während einer Abfolge aufeinander folgender Verabreichungen auch unterschiedliche Dosierungen eingesetzt werden.

Es ist zum Beispiel vorgesehen, dass Injektionen (intramuskulär oder subkutan oder in die Blutgefäße) ein Weg für die therapeutische Verabreichung der erfindungsgemäßen Verbindungen, beispielsweise der eingekapselten oder an Träger gebundenen erfindungsgemäßen Verbindungen, sind, obgleich die Zufuhr als Aerosol, über Katheter oder chirurgische Schläuche auch angewendet werden kann. Andere bevorzugte Wege umfassen Suspensionen, Tabletten, Kapseln und dergleichen für die orale Verabreichung, im Handel erhältliche Vernebler für flüssige Formulierungen und Inhalationen von lyophilisierten oder aerolysierten Verbindungen und Suppositorien für rektale oder vaginale Verabreichung. Flüssige Formulierungen können Die Eignung der gewählten Parameter, zum Beispiel Dosis, Schema, Adjuvanzwahl und dergleichen, kann durch Entnahme von Serum-Aliquoten aus dem Patienten - das heißt dem Mensch oder dem Tier - und Testen im Verlauf der Applikationen bestimmt werden. Alternativ und begleitend dazu kann die Menge von T-Zellen oder anderen Zellen des Immunsystems auf herkömmliche Weise bestimmt werden, um einen Gesamtüberblick über die immunologische Konstitution des Patienten zu erhalten. Zusätzlich kann der klinische Zustand des Patienten auf die gewünschte Wirkung hin beobachtet werden. Insbesondere kann das Wachstum und die Metastasierung von Tumoren bestimmt werden. Da Tumoren mit anderen Erkrankungen wie zum Beispiel Infektionen assoziiert sein können, ist es auch möglich, diese zusätzlich mit zu verfolgen.

Im Allgemeinen können sowohl wässrige Formulierungen als auch trockene erfindungsgemäße Verbindungen mit einem Exzipienten vermengt werden, um für eine stabilisierende Wirkung vor der Behandlung beispielsweise mit einem Lösungsmittel zu sorgen. Eine wässrige Lösung einer erfindungsgemäßen Verbindung kann eine erfindungsgemäße Verbindung in Suspension oder eine Lösung sein. Dem Fachmann sind weitere Zubereitungs-, Darreichungs- und Anwendungsformen bekannt, wie zum Beispiel als Gel, Puder, Pulver, Tablette, Retard-Tablette, Premix, Emulsion, Aufgussformulierung, Tropfen, Konzentrat, Granulat, Sirup, Pellet, Boli, Kapsel, Aerosol, Spray und/oder Inhalat. Die Behandlung der soliden Tumoren oder Leukämien umfasst Prophylaxe, Prävention, Diagnose, Verminderung, Therapie, Verlaufskontrolle und/oder Nachbehandlung einer Metastasierung, einer Invasion und/oder einer Angiogenese, wobei die Verlaufskontrolle eine Überwachung der Wirksamkeit einer Antitumorbehandlung ist.

Die erfindungsgemäße Verbindung kann in einer Lösung mit einem Konservierungsmittel eingebracht sein. Beispiele für geeignete Konservierungsmittel der Suspension bzw. Lösungen umfassen Phenol, Benzylalkohol, m-Kresol, Metylparaben, Propylparaben, Benzalkoniumchlorid und Benzethoniumchlorid. Im Allgemeinen können die Formulierungen der erfindungsgemäßen Verbindungen Komponenten in Mengen enthalten, die der Herstellung stabiler Formen nicht abträglich sind und Mengen, die für wirksame, sichere pharmazeutische Verabreichungen geeignet sind. Beispielsweise können andere pharmazeutisch annehmbare, dem Fachkundigen bekannte Exzipienten einen Teil der erfindungsgemäßen Verbindungen oder Formulierungen bilden. Diese umfassen beispielsweise Salze, verschiedene Füller, zusätzliche Pufferagenzien, Chelatbildner, Antioxydanzien, Co-Lösungsmittel und dergleichen.

In einer bevorzugten Ausführungsform der Erfindung sind die erfindungsgemäßen Verbindungen mit Liposomen, Siosomen und/oder Niosomen assoziiert.

Dies kann beispielsweise so erfolgen, dass die erfindungsgemäße Verbindung in einem Liposom eingeschlossen oder auf der Liposomenoberfläche verankert vorliegt. Dem Fachmann ist bekannt, dass künstliche bzw. natürliche Membranen von Liposomen eine immunstimulierende Wirkung haben können, insbesondere dann, wenn die Komponenten auf die Oberfläche der Liposomen gekoppelt werden oder im Inneren der Liposomen eingeschlossen sind oder einfach nur mit den Liposomen zusammen vermischt werden. Derartige Formulierungen von Liposomen können parentral appliziert werden. Es ist möglich, derartige Formulierungen mit bekannten Methoden, wie zum Beispiel einem Spray, nasal auf die Schleimhäute der Nasenhöhlen zu applizieren. Eine mit dem Spray vorgenommene therapeutische Behandlung ist bevorzugt für die Behandlung von Lungenkrebs oder Hals-Nasen-Ohren Tumoren geeignet. Insbesondere bei der nasalen Verabreichung muss die erfindungsgemäße Verbindung in einem solchen Zustand auf die Schleimhaut aufgetragen werden, dass sie in der Lage ist, die Schleimhaut zu durchdringen oder durch diese absorbiert zu werden. Daher muss das Vesikel mit dem Schleim biokompatibel sein und ein gewisses Maß an Hydrophilie aufweisen. Dem Fachmann sind derartige Strukturen beispielsweise aus der EP 0682528 bekannt, deren Lehre mit in den Offenbarungsgehalt der Erfindung aufgenommen ist. Die liposomale Zusammensetzung kann einen oder mehrere zusätzliche pharmazeutische Träger umfassen, die ausgewählt sind aus oberflächenaktiven Stoffen und Absorptionsförderern wie zum Beispiel Polyoxyethylenalkoholethern, Gallensalzen und deren Derivaten, Fusidinsäure und deren Derivaten, Oleinsäure, Lecithin, Lysolecithinen, Tween^{®} 21 bis 85, usw., wasserabsorbierenden Polymeren wie zum Beispiel Glycofurol, Polyethylenglycol 200 bis 7500, Polyvinylpyrrolidon, Propylenglycol oder Polyacrylsäure, Gelatine, Cellulose und Derivaten usw.; Substanzen, welche den enzymatischen Abbau hemmen, wie zum Beispiel Aprotinin usw.; organischen Lösungsmitteln wie zum Beispiel Alkoholen, zum Beispiel Ethanol, Glycerol, Benzylalkohol usw.; oder Ethylacetat usw.; hydrophoben Mitteln wie zum Beispiel pflanzlichem Öl, Sojabohnenöl, Erdnussöl, Kokosnussöl, Maisöl, Olivenöl, Sonnenblumenöl, "Miglyolen" oder deren Mischungen usw.; pH-regulierenden Mitteln wie zum Beispiel Salpetersäure, Phosphorsäure, Essigsäure, Citraten usw.; Konservierungsmitteln und den osmotischen Druck regulierenden Mitteln wie zum Beispiel Glycerol, Natriumchlorid, Methylparaoxybenzoat, Benzoesäure usw.; Liposomen- und/oder Emulsionsformulierungen wie zum Beispiel Lecithinen usw.; mikroverkapselten Formulierungen; Treibmitteln wie zum Beispiel Butan.

Bevorzugt ist es in einer weiteren Ausführungsform der Erfindung, dass die erfindungsgemäßen Verbindungen gegebenenfalls miteinander assoziiert oder mit einem Träger verbunden in Liposomen eingeschlossen sind, wobei der Einschluss in Liposomen im Sinne der Erfindung nicht zwingend bedeuten muss, dass die erfindungsgemäßen Verbindungen im Inneren der Liposomen vorliegen, ein Einschluss im Sinne der Erfindung kann auch bedeuten, dass die erfindungsgemäßen Verbindungen mit der Membran der Liposomen assoziiert sind, beispielsweise so, dass diese auf der äußeren Membran verankert sind. Eine solche Darstellung der erfindungsgemäßen Verbindungen in oder auf den Liposomen ist vorteilhaft, wenn der Fachmann die Liposomen so auswählt, dass sie eine immunstimulierende Wirkung haben. Dem Fachmann sind aus der DE 198 51 282 verschiedene Möglichkeiten bekannt, die immunstimulierende Wirkung von Liposomen zu modifizieren. Bei den Lipiden kann es sich um einfache Lipide handeln, wie beispielsweise Ester und Amide oder um komplexe Lipide wie zum Beispiel um Glycolipide wie Cerebroside oder Ganglioside, um Sphingolipide oder um Phospholipide.

Bei den Trägern, die Bestandteil von Arzneimitteln sein können - die die erfindungsgemäßen Verbindungen umfassen -, kann es sich im Sinne der Erfindung um Proteine handeln, die aufgrund ihres immunogenen Verhaltens die Antikörperantwort stimulieren, aber auch um den Fachmann bekannte pharmazeutische Hilfsstoffe wie zum Beispiel QS21, GPI-0100 oder andere Saponine, Wasser-Öl-Emulsionen wie beispielsweise Montanide, Polylysin, Polyagenin-Verbindungen oder andere, wie zum Beispiel phosphat-gepufferte Kochsalzlösung, Wasser, verschiedene Arten von Detergenzien, sterile Lösungen und dergleichen mehr.

Ein pharmazeutisches Mittel im Sinne der Erfindung ist jedes Mittel im Bereich der Medizin, welches in der Prophylaxe, Diagnose, Therapie, Verlaufskontrolle oder Nachbehandlung von Patienten eingesetzt werden kann, die einen Tumor so umfassen, dass sich zumindest zeitweise eine pathogene Modifikation des Gesamtzustandes bzw. des Zustandes einzelner Teile des Organismus etablieren konnte. So ist es beispielsweise möglich, dass das pharmazeutische Mittel im Sinne der Erfindung eine Vakzine oder ein Therapeutikum ist. Das pharmazeutische Mittel im Sinne der Erfindung kann neben den erfindungsgemäßen Verbindungen beispielsweise ein akzeptables Salz oder Komponenten dieser umfassen. Hierbei kann es sich beispielsweise um Salze anorganischer Säuren handeln wie zum Beispiel der Phosphorsäure bzw. um Salze organischer Säuren.

Weiterhin ist es möglich, dass die Salze frei von Carboxylgruppen sind und von anorganischen Basen abgeleitet wurden wie zum Beispiel Natrium, Kalium, Ammonium, Calcium oder Eisenhydroxyde oder auch von organischen Basen wie Isopropylamin, Trimethylamin, 2-Ethylaminoethanol, Histidin und andere. Beispiele für flüssige Träger sind sterile wässrige Lösungen, die keine weiteren Materialien oder aktiven Ingredienzien umfassen und beispielsweise Wasser oder solche, die einen Puffer wie zum Beispiel Natriumphosphat mit einem physiologischen pH umfassen oder eine physiologische Salzlösung bzw. beides, wie zum Beispiel phosphatgepufferte Natriumchloridlösung. Weitere flüssige Träger können mehr als nur ein Puffersalz, wie zum Beispiel Natrium- und Kaliumchlorid, Dextrose, Propylenglycol, Polyethylenglycol oder andere umfassen. Flüssige Zusammensetzungen der pharmazeutischen Mittel können zusätzlich eine flüssige Phase, jedoch unter dem Ausschluss von Wasser, umfassen. Beispiele solcher zusätzlichen flüssigen Phasen sind Glycerin, Pflanzenöle, organische Ester oder Wasser-Öl-Emulsionen. Die pharmazeutische Zusammensetzung bzw. das pharmazeutische Mittel enthält typischerweise einen Gehalt von mindestens 0,1 Gew% der erfindungsgemäßen Verbindungen bezogen auf die gesamte pharmazeutische Zusammensetzung. Die jeweilige Dosis bzw. der Dosisbereich für die Gabe des erfindungsgemäßen pharmazeutischen Mittels ist groß genug, um den gewünschten prophylaktischen oder therapeutischen Effekt der Bildung von neutralisierenden Antikörpern zu erreichen. Hierbei sollte die Dosis nicht so gewählt werden, dass unerwünschte Nebeneffekte dominieren. Im Allgemeinen wird die Dosis mit dem Alter, der Konstitution, dem Geschlecht des Patienten variieren sowie selbstverständlich auch in Bezug auf die Schwere der Erkrankung. Die individuelle Dosis kann sowohl in Bezug auf die primäre Erkrankung als auch in Bezug auf Eintreten zusätzlicher Komplikationen eingestellt werden. Die exakte Dosis ist durch einen Fachmann mit bekannten Mitteln und Methoden feststellbar, beispielsweise durch die Feststellung des Tumorwachstums in Abhängigkeit der Dosis bzw. in Abhängigkeit des Applikationsschemas oder der pharmazeutischen Träger und ähnlichem. Die Dosis kann hierbei je nach Patient individuell gewählt werden. Beispielsweise kann eine vom Patienten noch tolerierte Dosis des pharmazeutischen Mittels eine solche sein, deren Bereich im Plasma oder in einzelnen Organen lokal im Bereich von 0,1 bis 10000 µM liegt, bevorzugt zwischen 1 und 100 µM. Alternativ kann die Dosis auch in Bezug auf das Körpergewicht des Patienten bezogen berechnet werden. In einem solchen Fall wäre beispielsweise eine typische Dosis des pharmazeutischen Mittels in einem Bereich zwischen 0,1 µg bis 100 µg per kg Körpergewicht einzustellen, bevorzugt zwischen 1 und 50 µg/kg. Weiterhin ist es jedoch auch möglich, die Dosis nicht in Bezug auf den gesamten Patienten, sondern in Bezug auf einzelne Organe zu bestimmen. Dies wäre beispielsweise dann der Fall, wenn das erfindungsgemäße pharmazeutische Mittel beispielsweise in einem Biopolymer, eingebracht in den jeweiligen Patienten, in der Nähe bestimmter Organe mittels einer Operation platziert wird. Dem Fachmann sind mehrere Biopolymere bekannt, die Peptide oder rekombinante Proteine in einer gewünschten Art und Weise freisetzen können. Ein solches Gel kann beispielsweise 1 bis 1000 *µ*g der erfindungsgemäßen Aminosäuresequenzen, zum Beispiel Peptide oder rekombinanten Proteine bzw. des pharmazeutischen Mittels pro ml Gelkomposition beinhalten, bevorzugt zwischen 5 bis 500 µg/ml und besonders bevorzugt zwischen 10 und 100 mg/ml. In solch einem Fall wird das therapeutische Mittel als feste, gelartige oder als flüssige Komposition verabreicht.

In einer weiteren bevorzugten Ausführungsform der Erfindung sind die Träger ausgewählt aus der Gruppe umfassend Füllmittel, Streckmittel, Bindemittel, Feuchthaltemittel, Sprengmittel, Lösungsverzögerer, Resorptionsbeschleuniger, Netzmittel, Adsorbtionsmittel, und/oder Gleitmittel.

Hierbei handelt es sich bei den Füll- und Streckmitteln bevorzugt um Stärken, Milchzucker, Rohrzucker, Glukose, Mannit und Kieselsäure, bei dem Bindemittel, bevorzugt Carbocymethylcellulose, Alginate, Gelatine, Polyvinylpyrrolidon, bei dem Feuchthaltemittel bevorzugt um Glycerin, bei dem Sprengmittel bevorzugt um Agar-Agar, Calciumcarbonat und Natriumcarbonat, bei dem Lösungsverzögerer vorzugsweise um Paraffin und bei dem Resorptionsbeschleuniger bevorzugt um quarternäre Ammoniumverbindungen, bei dem Netzmittel bevorzugt um Cetylalkohol und Glycerinmonostearat, bei dem Adsorptionsmittel bevorzugt um Kaolin und Bentonit und bei dem Gleitmittel bevorzugt um Talkum, Calcium- und Magnesiumstearat und feste Polythylenglykole oder Gemische der aufgeführten Stoffe.

In einer weiteren bevorzugten Ausführungsform der Erfindung werden die erfindungsgemäßen Verbindungen als Gel, Puder, Pulver, Tablette, Retard-Tablette, Premix, Emulsion, Aufgussformulierung, Tropfen, Konzentrat, Granulat, Sirup, Pellet, Boli, Kapsel, Aerosol, Spray und/oder Inhalat zubereitet und/oder in dieser Form angewendet. Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, dass sie den oder die Wirkstoffe nur oder bevorzugt in einem bestimmten Teil des Intestinaltraktes gegebenenfalls verzögert abgeben, wobei als Einbettungsmassen zum Beispiel Polymersubstanzen und Wachse verwendet werden können.

Die Arzneimittel dieser Erfindung können bevorzugt zur oralen Verabreichung in einer beliebigen oral verträglichen Dosierungsform verwendet werden, die Kapseln, Tabletten und wässrige Suspensionen und Lösungen einschließt, aber nicht darauf beschränkt ist. Im Fall von Tabletten zur oralen Verwendung schließen Träger, die häufig verwendet werden, Lactose und Maisstärke ein. Gleitmittel, wie Magnesiumstearat, werden auch typischerweise zugesetzt. Zur oralen Verabreichung in Kapselform schließen verwendbare Verdünnungsmittel Lactose und getrocknete Maisstärke ein. Wenn wässrige Suspensionen oral verabreicht werden, wird der Wirkstoff mit Emulgier- und Suspendiermitteln kombiniert. Falls gewünscht, können bestimmte Süßmittel und/oder Geschmacksstoffe und/oder Farbmittel zugesetzt werden.

Der oder die Wirkstoffe - das heißt die erfindungsgemäßen Verbindungen - können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, zum Beispiel Polyethylenglycole, Fette, zum Beispiel Kakaofett und höhere Ester (zum Beispiel C₁₄₋Alkohol mit C₁₆-Fettsäure) oder Gemische dieser Stoffe).

Salben, Pasten, Cremes und Gele können neben dem oder den Wirkstoffen die üblichen Trägerstoffe enthalten, zum Beispiel tierische und pflanzliche Fette, Wachse, Paraffine, Stärke, Tragant, Cellulosederivate, Polyethylenglycole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, zum Beispiel Milchzucker, Talkum, Kieselsäure, Aluminiumhydroxid, Calciumsilikat und Polyamidpulver oder Gemische dieser Stoffe. Sprays können zusätzlich die üblichen Treibmittel, zum Beispiel Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, zum Beispiel Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnussöl, Maiskeimöl, Olivenöl, Ricinusöl und Sesamöl, Glycerin, Glycerinformal, Tetrahydofurfurylalkohol, Polyethylenglycole und Fettsäureester des Sorbitans oder Gemische dieser Stoffe enthalten. Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, zum Beispiel Wasser, Ethylalkohol, Propylenglykol, Suspendiermittel, zum Beispiel ethoxilierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die Arzneimittel können in Form einer sterilen injizierbaren Zubereitung, zum Beispiel als sterile injizierbare wässrige oder ölige Suspension, vorliegen. Diese Suspension kann auch im Fachgebiet bekannten Verfahren unter Verwendung geeigneter Dispergier- oder Netzmittel (wie zum Beispiel Tween 80) und Suspendiermittel formuliert werden. Die sterile injizierbare Zubereitung kann auch eine sterile injizierbare Lösung oder Suspension in einem ungiftigen parenteral verträglichen Verdünnungs- oder Lösungsmittel, zum Beispiel als Lösung in 1,3-Butandiol, sein. Zu den verträglichen Vehikeln und Lösungsmitteln, die verwendet werden können, gehören Mannit, Wasser, Ringer-Lösung und isotonische Natriumchloridlösung. Außerdem werden üblicherweise sterile, nichtflüchtige Öle als Lösungsmittel oder Suspendiermedium verwendet. Zu diesem Zweck kann ein beliebiges mildes nichtflüchtiges Öl einschließlich synthetischer Mono- oder Diglyceride verwendet werden. Fettsäuren, wie Ölsäure und ihre Glyceridderivate sind bei der Herstellung von Injektionsmitteln verwendbar, wie es natürliche pharmazeutisch verträgliche Öle, wie Olivenöl oder Rizinusöl, insbesondere in ihren polyoxyethylierten Formen sind. Diese Öllösungen oder Suspensionen können auch einen langkettigen Alkohol oder einen ähnlichen Alkohol enthalten als Verdünnungs- oder Dispergiermittel.

Die genannten Formulierungsformen können auch Färbemittel, Konservierungsstoffe sowie geruchs- und geschmacksverbesserte Zusätze, zum Beispiel Pfefferminzöl und Eukalyptusöl und Süßmittel, zum Beispiel Saccharin, enthalten. Die erfindungsgemäßen Verbindungen sollen in den aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die aufgeführten pharmazeutischen Zubereitungen können außer den erfindungsgemäßen Verbindungen auch weitere pharmazeutische Wirkstoffe enthalten. Die Herstellung der oben aufgeführten pharmazeutischen Zubereitungen erfolgt in üblicher Weise nach bekannten Methoden, zum Beispiel durch Mischen des oder der Wirkstoffe mit dem oder den Trägerstoffen.

Die genannten Zubereitungen können bei Mensch und Tier entweder oral, nasal, rektal, regional - zum Beispiel Leber, Milz, Niere, Lunge oder ähnliches - parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitung kommen Injektionslösungen, Lösungen und Suspensionen für die orale Therapie, Gele, Aufgussformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Tabletten, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

In einer weiteren bevorzugten Ausführungsform der Erfindung sind die erfindungsgemäßen Verbindungen in einer Konzentration von 0,1 bis 99,5, bevorzugt von 0,5 bis 95, besonders bevorzugt von 20 bis 80 Gew.-% in einer Zubereitung eingebracht. Das heißt, die erfindungsgemäßen Verbindungen sind in den oben aufgeführten pharmazeutischen Zubereitungen, zum Beispiel Tabletten, Pillen, Granulaten und anderen, vorzugsweise in einer Konzentration von 0,1 bis 99,5 Gew.-% der Gesamtmischung vorhanden. Die Wirkstoffmenge, das heißt die Menge einer erfindungsgemäßen Verbindung, die mit den Trägermaterialien kombiniert wird, um eine einzige Dosierungsform zu erzeugen, wird von dem Fachmann in Abhängigkeit von dem zu behandelnden Wirt, vom zu behandelnden Tumor und der besonderen Verabreichungsart variieren können. Nach Besserung des Zustandes eines Wirts bzw. eines Patienten kann der Anteil der wirksamen Verbindung in der Zubereitung so geändert werden, dass eine Erhaltungsdosis vorliegt. Anschließend kann die Dosis oder Frequenz der Verabreichung oder beides als Funktion der Symptome auf eine Höhe verringert werden, bei der der verbesserte Zustand beibehalten wird. Wenn die Symptome auf das gewünschte Niveau gelindert worden sind, sollte die Behandlung aufhören. Patienten können jedoch eine Behandlung mit Unterbrechung auf Langzeitbasis nach beliebigem Wiederauftreten von Erkrankungssymptomen benötigen. Demgemäß ist der Anteil der Verbindungen, das heißt ihre Konzentration, in der Gesamtmischung der pharmazeutischen Zubereitung ebenso wie ihre Zusammensetzung oder Kombination variabel und kann vom Fachmann aufgrund seines Fachwissens modifiziert und angepasst werden.

Dem Fachmann ist bekannt, dass die erfindungsgemäßen Verbindungen mit einem Organismus, bevorzugt einem Menschen oder einem Tier, auf verschiedenen Wegen in Kontakt gebracht werden können. Weiterhin ist dem Fachmann bekannt, dass insbesondere die pharmazeutischen Mittel in verschiedenen Dosierungen appliziert werden können. Die Applikation sollte hierbei so erfolgen, dass der Tumor möglichst effektiv bekämpft wird bzw. der Ausbruch einer solchen Krankheit in einer prophylaktischen Gabe verhindert wird. Die Konzentration und die Art der Applikation kann vom Fachmann durch Routineversuche eruiert werden. Bevorzugte Applikationen der erfindungsgemäßen Verbindungen sind die orale Applikation in Form von Pulver, Tabletten, Saft, Tropfen, Kapseln oder ähnlichem, die rektale Applikation in Form von Zäpfchen, Lösungen und ähnlichem, parenteral in Form von Injektionen, Infusionen und Lösungen, Inhalation von Dämpfen, Aerosolen und Stäuben und Pflastern sowie lokal in Form von Salben, Pflastern, Umschlägen, Spülungen und ähnlichem. Bevorzugt erfolgt das In-Kontakt-Bringen der erfindungsgemäßen Verbindungen prophylaktisch oder therapeutisch. Bei einer prophylaktischen Gabe soll die Bildung den genannten Tumoren zumindest dergestalt verhindert werden, dass eine weitere Vermehrung dieser stark vermindert wird oder dass Tumoren nahezu vollständig eliminiert werden. Bei einem therapeutischen In-Kontakt-Bringen liegt bereits eine Tumorerkrankung des Patienten vor und die bereits im Körper vorhandenen Tumoren sollen entweder abgetötet oder in ihrer Vermehrung gehemmt werden. Weitere hierfür bevorzugte Applikationsformen sind beispielsweise die subkutane, die sublinguale, die intravenöse, die intramuskuläre, die intraperitoneale und/oder die topische.

Neben den bereits ausgeführten Konzentrationen bei der Anwendung der erfindungsgemäßen Verbindungen können in einer bevorzugten Ausführungsform die Verbindungen weiterhin in einer Gesamtmenge von 0,05 bis 500 mg/kg Körpergewicht je 24 Stunden eingesetzt werden, bevorzugt von 5 bis 100 mg/kg Körpergewicht. Hierbei handelt es sich vorteilhafterweise um eine therapeutische Menge, die verwendet wird, um die Symptome einer Störung oder responsiven, pathologisch physiologischen Kondition zu verhindern oder zu verbessern. Die verabreichte Menge ist ausreichend, um das Tumorwachstum zu hemmen.

Selbstverständlich wird die Dosis vom Alter, der Gesundheit und dem Gewicht des Empfängers, dem Grad der Krankheit, der Art einer notwendigen gleichzeitigen Behandlung, der Häufigkeit der Behandlung und der Art der gewünschten Wirkungen und der Nebenwirkungen abhängen. Die tägliche Dosis von 0,05 bis 500 mg/kg Körpergewicht kann einmalig oder mehrfach angewendet werden, um die gewünschten Ergebnisse zu erhalten. Die Dosishöhen pro Tag sind bei der Vorbeugung und bei der Behandlung einer Tumorerkrankung einschließlich einer Infektion anwendbar, beispielsweise bei Infektionen, die einen Tumor verursachen oder mit verursachen können, wie zum Beispiel eine Hepatitis-, insbesondere eine Hepatitis B-Infektion. Typischerweise werden insbesondere pharmazeutischen Mittel zur etwa 1- bis 7-maligen Verabreichung pro Tag oder alternativ oder zusätzlich als kontinuierliche Infusion verwendet. Solche Verabreichungen können als chronische oder akute Therapie angewendet werden. Die Wirkstoffmengen, die mit den Trägermaterialien kombiniert werden, um eine einzelne Dosierungsform zu erzeugen, können in Abhängigkeit von dem zu behandelnden Wirt und der besonderen Verabreichungsart selbstverständlich variieren. Bevorzugt ist es, die Targetsdosis auf 2 bis 5 Applikationen zu verteilen, wobei bei jeder Applikation 1 bis 2 Tabletten mit einem Wirkstoffgehalt von 0,05 bis 500 mg/kg Körpergewicht verabreicht werden. Selbstverständlich ist es möglich, den Wirkstoffgehalt auch höher zu wählen, beispielsweise bis zu einer Konzentration bis 5000 mg/kg. Beispielsweise können Tabletten auch retardiert sein, wodurch sich die Anzahl der Applikationen pro Tag auf 1 bis 3 vermindert. Der Wirkstoffgehalt der retardierten Tabletten kann 3 bis 3000 mg betragen. Wenn der Wirkstoff - wie ausgeführt - durch eine Injektion verabreicht wird, ist es bevorzugt, 1- bis 8-mal pro Tag bzw. durch Dauerinfusion den Wirt mit den erfindungsgemäßen Verbindungen in Kontakt zu bringen, wobei Mengen von 4 bis 4000 mg pro Tag bevorzugt sind. Die bevorzugten Gesamtmengen pro Tag haben sich in der Humanmedizin und in der Veterinärmedizin als vorteilhaft erwiesen. Es kann erforderlich sein, von den genannten Dosierungen abzuweichen und zwar in Abhängigkeit von der Art und dem Körpergewicht des zu behandelnden Wirts, der Art und der Schwere der Erkrankung, der Art der Zubereitung der Applikation des Arzneimittels sowie dem Zeitraum bzw. dem Intervall, innerhalb welchem die Verabreichung erfolgt. So kann es in einigen Fällen bevorzugt sein, den Organismus mit weniger als den genannten Mengen in Kontakt zu bringen, während in anderen Fällen die angegebene Wirkstoffmenge überschritten werden muss. Die Festlegung der jeweils erforderlichen optimalen Dosierungen und der Applikationsart der Wirkstoffe kann durch den Fachmann aufgrund seines Fachwissens leicht erfolgen.

In einer weiteren besonders bevorzugten Ausführungsform der Erfindung werden die erfindungsgemäßen Verbindungen in einer Einzelgabe von 1 bis 80, insbesondere von 3 bis 30 mg/kg Körpergewicht eingesetzt. Wie auch die Gesamtmenge pro Tag kann auch die Menge der Einzelgabe pro Applikation von dem Fachmann aufgrund seines Fachwissens variiert werden. Die erfindungsgemäß verwendeten Verbindungen können in den genannten Einzelkonzentrationen und Zubereitungen zusammen mit dem Futter bzw. mit Futterzubereitungen oder mit dem Trinkwasser auch in der Veterinärmedizin gegeben werden. Eine Einzeldosis enthält vorzugsweise die Menge Wirkstoff, die bei einer Applikation verabreicht wird, und die gewöhnlich einer ganzen, einer halben Tagesdosis oder einem Drittel oder einem Viertel einer Tagesdosis entspricht. Die Dosierungseinheiten können demgemäß bevorzugt 1, 2, 3 oder 4 oder mehrere Einzeldosen oder 0,5, 0,3 oder 0,25 einer Einzeldosis enthalten. Bevorzugt wird die Tagesdosis der erfindungsgemäßen Verbindungen auf 2 bis 10 Applikationen verteilt, bevorzugt auf 2 bis 7, besonders bevorzugt auf 3 bis 5 Applikationen. Selbstverständlich ist auch eine Dauerinfusion der erfindungsgemäßen Mittel möglich.

In einer besonders bevorzugten Ausführungsform der Erfindung werden bei jeder oralen Applikation der erfindungsgemäßen Verbindungen 1 bis 10 Tabletten oder Kapseln gegeben, bevorzugt 4 bis 8 Kapseln oder Tabletten und ganz besonders bevorzugt 6 Kapseln oder Tabletten. Die erfindungsgemäßen Tabletten oder Kapseln können mit dem Fachmann bekannten Überzügen und Hüllen versehen sein und auch so zusammengesetzt werden, dass sie den oder die Wirkstoffe nur bei bevorzugten, in einem bestimmten Teil des Wirts freigeben.

In einer weiteren bevorzugten Ausführungsform der Erfindung können die erfindungsgemäßen Verbindungen mit mindestens einem anderen bekannten pharmazeutischen Mittel eingesetzt werden. Das heißt, die erfindungsgemäßen Verbindungen können in einer prophylaktischen oder therapeutischen Kombination in Verbindung mit den bekannten Arzneimitteln eingesetzt werden. Diese Kombinationen können gemeinsam, zum Beispiel in einer einheitlichen pharmazeutischen Formulierung verabreicht werden, oder getrennt, zum Beispiel in Form einer Kombination aus Tabletten, Injektion oder anderen Medikamenten, die zu gleichen oder zu unterschiedlichen Zeiten verabreicht werden, mit dem Ziel, die gewünschte prophylaktische oder therapeutische Wirkung zu erzielen. Bei diesen bekannten Mitteln kann es sich um Mittel handeln, die die Wirkung der erfindungsgemäßen Verbindungen steigern.

Selbstverständlich ist es auch möglich, die erfindungsgemäßen Verbindungen, insbesondere die pharmazeutischen Mittel, allein oder zusammen mit anderen Mitteln in einer Therapie, beispielsweise in einer Kombinationstherapie, als regionale Therapie einzusetzen; dies kann beispielsweise bei einem Lebertumor bevorzugt sein.

Dem Fachmann ist bekannt, dass es vorteilhaft sein kann, bei bestimmten Tumorerkrankungen die Konzentration von Oxidanzien von Glutation oder die Inaktivierung von Glutation zu erhöhen. Es kann zur Glutationabreicherung zum Beispiel DL-Buthionin-SR-Sulfoximin eingesetzt werden.

Typischerweise gibt es ein optimales Verhältnis der erfindungsgemäßen Verbindung(en) untereinander und/oder mit anderen therapeutischen oder wirkungssteigernden Mitteln (wie Transportinhibitoren und/oder -aktivatoren, Stoffwechselinhibitoren, Inhibitoren oder Aktivatoren für die Nierenausscheidung oder Glukuronidation, wie Probenecid, Acetaminophen, Aspirin, Lorazepam, Cimetidin, Ranitidin, Colifibrat, Indomethacin, Ketoprofen, Naproxen etc.) in dem die Wirkstoffe in einem optimalen Verhältnis vorliegen. Ein optimales Verhältnis ist als das Verhältnis definiert, bei dem die erfindungsgemäße(n) Verbindung(en) mit einem anderen therapeutischen Mittel oder Mitteln so ist, dass die therapeutische Gesamtwirkung größer ist als die Summe der Wirkungen der einzelnen therapeutischen Mittel. Das optimale Verhältnis findet man üblicherweise, wenn die Mittel im Verhältnis von 10:1 bis 1:10, 20:1 bis 1:20, 100:1 bis 1:100 und 500:1 bis 1:500 vorliegen. Gelegentlich wird eine verschwindend geringe Menge eines therapeutischen Mittels genügen, um die Wirkung eines oder mehrerer anderer Mittel zu steigern. Zusätzlich ist die Verwendung der erfindungsgemäßen Verbindungen in Kombinationen besonders nützlich, um das Risiko der Resistenzentwicklung herabzusetzen und/oder die therapeutische Wirksamkeit zu erhöhen. Selbstverständlich können die erfindungsgemäßen Verbindungen in Kombination mit anderen bekannten anti-Tumor Mitteln verwendet werden. Dem Fachmann sind derartige Mittel bekannt. Die erfindungsgemäßen Verbindungen können demgemäß mit allen herkömmlichen Mitteln, insbesondere anderen Arzneimitteln, verabreicht werden, die für die Verwendung im Zusammenhang insbesondere mit Tumor-Arzneimitteln verfügbar sind, entweder als einzelne Arzneimittel oder in Kombination von Arzneimitteln. Sie können allein verabreicht werden oder in Kombination mit diesen.

Bevorzugt ist es, dass die erfindungsgemäßen Verbindungen mit den anderen bekannten pharmazeutischen Mitteln im Verhältnis von etwa 0,005 zu 1 verabreicht werden. Bevorzugt ist es, die erfindungsgemäßen Verbindungen mit insbesondere virushemmenden Mitteln im Verhältnis von 0,05 bis etwa 0,5 Teilen zu etwa 1 Teil der bekannten Mittel zu verabreichen. Die pharmazeutische Zusammensetzung kann in Substanz oder als wässrige Lösung vorliegen zusammen mit anderen Materialen wie Konservierungsmitteln, Puffersubstanzen, Mitteln die zur Einstellung der Osmolarität der Lösung vorgesehen sind etc.

Bevorzugt wird das pharmazeutische Mittel auch als Vakzine nach der Etablierung eines Tumors oder als vorbeugende Impfung eingesetzt. Die Impfung erfolgt vorteilhafterweise so, dass es nach der Applikation im Organismus zur Entwicklung eines Schutzes gegen die Ausbreitung oder die Bildung von Tumoren kommt. Selbstverständlich ist es auch möglich, dass die Impfung unmittelbar vor oder zeitnah nach der Manifestation eines Tumors erfolgt oder als Therapie mehrfach appliziert wird. Dem Fachmann ist bekannt, dass die Behandlung eines Tumors zu nahezu jedem Zeitpunkt auch nach der Bildung von Metastasen von Vorteil sein kann, so dass eine Impfung im Sinne der Erfindung auch eine Applikation des erfindungsgemäßen pharmazeutischen Mittels Wochen, Monate, Jahre bzw. Jahrzehnte nach der Bildung eines Tumors sein kann.

Die Erfindung betrifft auch einen Kit und dessen Verwendung in der Medizin. Es ist bevorzugt, die erfindungsgemäßen Verbindungen oder den Kit, der diese umfasst, in einer Kombinationstherapie, insbesondere zur Behandlung von Tumoren, zu verwenden. Besonders bevorzugt ist hierbei, dass die Kombinationstherapie eine Chemotherapie, eine Zytostatikabehandlung und/oder eine Strahlentherapie umfasst. In einer besonders bevorzugten Ausführungsform der Erfindung ist die Kombinationstherapie eine adjuvante biologischspezifizierte Therapieform. Ganz besonders bevorzugt ist hierbei, dass diese Therapieform eine Immuntherapie ist. Weiterhin ist besonders bevorzugt, dass die Kombinationstherapie eine Gentherapie und/oder eine Therapie mit einer erfindungsgemäßen Verbindung umfasst. Dem Fachmann sind verschiedene Kombinationstherapien, insbesondere zur Behandlung von Tumoren, bekannt. Es kann zum Beispiel vorgesehen sein, dass innerhalb einer Kombinationstherapie eine Zytostatikabehandlung erfolgt oder beispielsweise eine Bestrahlung eines bestimmten Tumorareals, wobei diese Behandlung mit einer Gentherapie kombiniert wird, wobei die erfindungsgemäßen Verbindungen als Antikrebsmittel eingesetzt werden. Demgemäß kann es ganz besonders bevorzugt sein, dass die erfindungsgemäßen Verbindungen zur Erhöhung der Sensitivität von Tumorzellen gegenüber Zytostatika und/oder Strahlen verwendet werden. Weiterhin ist es bevorzugt, dass die erfindungsgemäßen Verbindungen zur Hemmung der Vitalität, der Proliferationsrate von Zellen und/oder zur Induktion von Apoptose und eines Zellzyklus-Arrests verwendet werden.

In einer bevorzugten Ausführungsform ist die Krebserkrankung oder der Tumor, die/der behandelt oder verhindert wird, ausgewählt aus der Gruppe von Krebserkrankungen oder Tumorerkrankungen des Hals-Nasen-Ohren-Bereichs, der Lunge, des Mediastinums, des Gastrointestinaltraktes, des Urogenitalsystems, des gynäkologischen Systems, der Brust, des endokrinen Systems, der Haut, Knochen- und Weichteilsarkomen, Mesotheliomen, Melanomen, Neoplasmen des zentralen Nervensystems, Krebserkrankungen oder Tumorerkrankungen im Kindesalter, Lymphomen, Leukämien, paraneoplastischen Syndromen, Metastasen ohne bekannten Primärtumor (CUP-Syndrom), peritonealen Karzinomastosen, Immunsuppressionbezogenen Malignitäten und/oder Tumor-Metastasen.

Insbesondere kann es sich bei den Tumoren um folgende Krebsarten handeln: Adenokarzinom der Brust, der Prostata und des Dickdarms; alle Formen von Lungenkrebs, der von den Bronchien ausgeht; Knochenmarkkrebs, das Melanom, das Hepatom, das Neuroblastom; das Papillom; das Apudom, das Choristom, das Branchiom; das maligne Karzinoid-Syndrom; die Karzinoid-Herzerkrankung; das Karzinom (zum Beispiel Walker-Karzinom, Basalzellen-Karzinom, basosquamöses Karzinom, Brown-Pearce-Karzinom, duktales Karzinom, Ehrlich-Tumor, in situ-Karzinom, Krebs-2-Karzinom, Merkel-Zellen-Karzinom, Schleimkrebs, nicht-kleinzelliges Bronchialkarzinom, Haferzellen-Karzinom, papilläres Karzinom, szirrhöses Karzinom, bronchiolo-alveoläres Karzinom, Bronchiai-Karzinom, Plattenepithelkarzinom und Transitionalzell-Karzinom); histiocytische Funktionsstörung; Leukämie (zum Beispiel in Zusammenhang mit B-Zellen-Leukämie, Gemischt-Zellen-Leukämie, Nullzellen-Leukämie, T-Zellen-Leukämie, chronische T-Zellen-Leukämie, HTLV-II-assoziierte Leukämie, akut lymphozytische Leukämie, chronisch-lymphozythische Leukämie, Mastzell-Leukämie und myeloische Leukämie); maligne Histiocytose, Hodgkin-Krankheit, non-Hodgkin-Lymphom, solitärer Plasmazelltumor; Reticuloendotheliose, Chondroblastom; Chondrom, Chondrosarkom; Fibrom; Fibrosarkom; Riesenzell-Tumore; Histiocytom; Lipom; Liposarkom; Leukosarkom; Mesotheliom; Myxom; Myxosarkom; Osteom; Osteosarkom; Ewing-Sarkom; Synoviom; Adenofribrom; Adenolymphom; Karzinosarkom, Chordom, Craniopharyngiom, Dysgerminom, Hamartom; Mesenchymom; Mesonephrom, Myosarkom, Ameloblastom, Cementom; Odontom; Teratom; Thymom, Chorioblastom; Adenokarzinom, Adenom; Cholangiom; Cholesteatom; Cylindrom; Cystadenocarcinom, Cystadenom; Granulosazelltumor; Gynadroblastom; Hidradenom; Inselzelltumor; Leydig-Zelltumor; Papillom; Sertoli-Zell-Tumor, Thekazelltumor, Leiomyom; Leiomyosarkom; Myoblastom; Myom; Myosarkom; Rhabdomyom; Rhabdomyosarkom; Ependynom; Ganglioneurom, Gliom; Medulloblastom, Meningiom; Neurilemmom; Neuroblastom; Neuroepitheliom, Neurofibrom, Neurom, Paragangliom, nicht-chromaffines Paragangliom, Angiokeratom, angiolymphoide Hyperplasie mit Eosinophilie; sclerosierendes Angiom; Angiomatose; Glomangiom; Hemangioendotheliom; Hemangiom; Hemangiopericytom, Hemangiosarkom; Lymphangiom, Lymphangiomyom, Lymphangiosarkom; Pinealom; Cystosarkom phyllodes; Hemangiosarkom; Lymphangiosarkom; Myxosarkom, Ovarialkarzinom; Sarkom (zum Beispiel Ewing-Sarkom, experimentell, Kaposi-Sarkom und Mastzell-Sarkom); Neoplasmen (zum Beispiel Knochen-Neoplasmen, Brust-Neoplasmen, Neoplasmen des Verdauungssystems, colorektale Neoplasmen, Leber-Neoplasmen, Pankreas-Neoplasmen, Hirnanhang-Neoplasmen, Hoden-Neoplasmen, Orbita-Neoplasmen, Neoplasmen des Kopfes und Halses, des Zentralnervensystems, Neoplasmen des Hörorgans, des Beckens, des Atmungstrakts und des Urogenitaltrakts); Neurofibromatose und zervikale Plattenepitheldysplasie.

In einer weiter bevorzugten Ausführungsform ist die Krebserkrankung oder der Tumor, die/der behandelt oder verhindert wird, ausgewählt aus der Gruppe: Tumoren des Hals-Nasen-Ohren-Bereichs umfassend Tumoren der inneren Nase, der Nasennebenhöhlen, des Nasopharynx, der Lippen, der Mundhöhle, des Oropharynx, des Larynx, des Hypopharynx, des Ohres, der Speicheldrüsen und Paragangliome, Tumoren der Lunge umfassend nicht-kleinzellige Bronchialkarzinome, kleinzellige Bronchialkarzinome, Tumoren des Mediastinums, Tumoren des Gastrointestinaltraktes umfassend Tumoren des Ösophagus, des Magens, des Pankreas, der Leber, der Gallenblase und der Gallenwege, des Dünndarms, Kolon- und Rektumkarzinome und Analkarzinome, Urogenitaltumoren umfassend Tumoren der Nieren, der Harnleiter, der Blase, der Prostata, der Harnröhre, des Penis und der Hoden, gynäkologische Tumoren umfassend Tumoren des Zervix, der Vagina, der Vulva, Korpuskarzinom, maligne Trophoblastenerkrankung, Ovarialkarzinom, Tumoren des Eileiters (Tuba Faloppii), Tumoren der Bauchhöhle, Mammakarzinome, Tumoren endokriner Organe umfassend Tumoren der Schilddrüse, der Nebenschilddrüse, der Nebennierenrinde, endokrine Pankreastumoren, Karzinoidtumoren und Karzinoidsyndrom, multiple endokrine Neoplasien, Knochen- und Weichteilsarkome, Mesotheliome, Hauttumoren, Melanome umfassend kutane und intraokulare Melanome, Tumoren des zentralen Nervensystems, Tumoren im Kindesalter umfassend Retinoblastom, Wilms Tumor, Neurofibromatose, Neuroblastom, Ewing-Sarkom Tumorfamilie, Rhabdomyosarkom, Lymphome umfassend Non-Hodgkin-Lymphome, kutane T-Zell-Lymphome, primäre Lymphome des zentralen Nervensystems, Morbus Hodgkin, Leukämien umfassend akute Leukämien, chronische myeloische und lymphatische Leukämien, Plasmazell-Neoplasmen, myelodysplastische Syndrome, paraneoplastische Syndrome, Metastasen ohne bekannten Primärtumor (CUP-Syndrom), peritoneale Karzinomastose, Immunsuppression-bezogene Malignität umfassend AIDS-bezogene Malignitäten wie Kaposi-Sarkom, AIDS-assoziierte Lymphome, AIDS-assoziierte Lymphome des zentralen Nervensystems, AIDS-assoziierter Morbus Hodgkin und AIDS-assoziierter anogenitale Tumoren, Transplantations-bezogene Malignitäten, metastasierte Tumoren umfassend Gehirnmetastasen, Lungenmetastasen, Lebernetastasen, Knochenmetastasen, pleurale und perikardiale Metastasen und maligne Aszites.

In einer weiter bevorzugten Ausführungsform ist die Krebserkrankung oder der Tumor, die/der behandelt oder verhindert wird, ausgewählt aus der Gruppe umfassend Krebserkrankungen oder Tumorerkrankungen der Mammakarzinome, der Gastrointestinaltumore, einschließlich Kolonkarzinome, Magenkarzinome, Pankreaskarzinome, Dickdarmkrebs, Dünndarmkrebs, der Ovarialkarzinome, der Zervikalkarzinome, Lungenkrebs, Prostatakrebs, Nierenzellkarzinome und/oder Lebermetastasen.

Im Folgenden soll die Erfindung anhand von Beispielen näher erläutert werden, ohne auf diese beschränkt zu sein.

### 1. Synthese von cis-Diammoniumdichlorodihydroxoplatin (IV)

150 g cis-Platin werden in einem 2 1-Dreihalskolben (versehen mit einem KPC-Rührer) in ca. 300 ml Wasser suspendiert und unter Rühren innerhalb von 2 Tagen portionsweise mit 350 ml H₂O₂ (35%ig) versetzt. Es wird anschließend noch 4 Tage bei (ca. 20 °C) weitergerührt. Danach wird das Rohprodukt abgesaugt, mit wenig eiskaltem Wasser gewaschen, in einer gerade ausreichenden Menge 0,5 N Schwefelsäure gelöst und dann mit 35%iger NaOH wieder ausgefällt (pH ~ 7 - 8) ; nach Aufbewahrung im Kühlschrank über Nacht wird die gelbe kristalline Masse abgesaugt, mit wenig kaltem Wasser, Ethanol und schließlich Ether gewaschen und im Vakuum getrocknet.
Ausbeute (3 Ansätze): 336,7 g (67,2 % d. Th.)

### 2. Synthese von cis-Diammoniumdichlorodihydroxoplatin (IV) Dinatriumsalz

20 g (0,06 Mol) cis-Diammoniumdichlorodihydroxoplatin (IV) werden in der Hitze in ca. 450 ml wässriger Natronlauge (enthalten 4,8 g (0,12 Mol) NaOH) gelöst und nach dem Abkühlen auf Raumtemperatur über Nacht im Kühlschrank aufbewahrt. Das ausgefallene Reaktionsprodukt wird abgesaugt, mit wenig kaltem Wasser, Ethanol und schließlich Ether gewaschen und im Vakuum getrocknet.
Ausbeute: 13,6 g (59,9 % d. Th.)

### 3. Anwendung von cis-Diammoniumdichlorodihydroxoplatin (IV) und seinen Salzen

Wachstumsinhibitionsversuche an verschiedenen humanen Zelllinien zeigen die unterschiedlichen Aktivitäten von cis-Platin, cis-Oxoplatin und Oxaliplatin. Die nachfolgend aufgeführten Ergebnisse zeigen, dass cis-Oxoplatin eine ähnliche Aktivität wie Oxaliplatin aufweist, aber eine größere Aktivität als Carboplatin besitzt. Die folgende Tabelle gibt die Ergebnisse wieder, die mit cis-Platin, Oxoplatin, Carboplatin und Oxaliplatin generiert wurden (die angegebenen Werte sind IC50-Werte in *µ*g/ml, das heißt, die Konzentration, bei der 50 % der Zellen überleben, nd = nicht bestimmt, res = resistent = nicht-sensitiv oder ein IC50-Wert kann bei einer Konzentration bis zu 40 *µ*g/ml nicht bestimmt werden; IC = Inhibitions-Konzentration).

**Tabelle 1**

| **Zelllinie** | **cis-Platin** | **cis-Oxoplatin** | **Carboplatin** | **Oxaliplatin** |
|---|---|---|---|---|
| HOS Osteosarcoma | nd | 2.5 | 5 | nd |
| SaOS Osteosarcoma | nd | 5 | 5 | nd |
| PC3 Prostate | res | 7.5 | 10 | nd |
| M607 Melanoma | 0.3 | 5 | 10 | 10 |
| M518 Melanoma | 40 | res | res | res |
| Me128 Melanoma | 0.3 | 2.5 | 10 | 10 |
| JVSO Melanoma | 40 | 10 | res | res |
| Panc1 Pancreatic cancer | 1 | 40 | 20 | 5 |
| BxPC3 Pancreatic cancer | 0.6 | 2.5 | 10 | 10 |
| MiaPaCa2 Pancreatic cancer | 1.5 | 5 | 5 | 5 |
| HCT8 Colon carcinoma | 5 | 40 | res | res |
| HT29 Colon carcinoma | 0.3 | 20 | 20 | 20 |
| HCT-15 Colon carcinoma | 0.3 | 20 | res | 10 |
| A498 Renal cells | 1 | 20 | res | 10 |
| C320DM Colon carcinoma | 0.3 | 2.5 | 10 | 0.15 |
| Colo205 Colon carcinoma | 10 | res | res | 1 |
| CC1227 Colon carcinoma | 0.3 | 10 | res | 0.2 |
| MCF-7 Brust cancer | 2.5 | 5.5 | res | res |
| T47D Brust cancer | 0.3 | 2.5 | nd | 0.1 |

**Tabelle 2**

| **Zelllinie** | **IC50 µg/ml** | |
|---|---|---|
| | **cis-Oxoplatin** | **Cis-Oxoplatin-Natriumsalz** |
| T47D Brust cancer | 3 | 18 |
| SK-OV3 Ovarian cancer | 15 | 22 |
| U 373 MG Astrocytoma | 15 | 18 |
| BxPC3 Pancreatic carcinoma | 13 | 12 |

Die ermittelten Ergebnisse zeigen, dass chemisch sehr ähnliche Platinverbindungen, wie zum Beispiel cis-Platin und cis-Oxoplatin, unterschiedliche Wirkungen auf verschiedene Humankrebszellen haben und dass die Salze der Platinverbindungen ein anderes Verhalten auf Tumoren zeigen als die Basenverbindungen, aus denen die Salze generiert wurden. Ganz allgemein und über die konkreten Versuche hinausgehend zeigt sich, dass die DNA-Bindungsfähigkeiten von cis-Oxoplatin-Salzen, insbesondere von cis-Oxoplatin-Natriumsalz, unerwartet gegenüber denen von cis-Oxoplatin sind. Dies kann beispielsweise seine Ursache in der unterschiedlichen Struktur der DNA-Addukte haben, die einerseits mit der Base und zum anderen mit dem Salz gebildet werden. Weiterhin kann vermutet werden, dass die cis-Oxoplatin-Salze einen anderen Prozess der Biotransformation durchlaufen als die entsprechenden Basen. Diese unerwarteten Unterschiede haben eine große Bedeutung beim Einsatz in der Tumortherapie von Basen und Salzen. Weitere wichtige Punkte des unterschiedlichen Verhaltens von Basen und Salzen sind zum Beispiel: die Aufnahme, die Ausbreitung und die Verteilung im Gewebe sowie in einzelnen Organen. Die intrazelluläre Aufnahme und die Toxizität der cis-Oxoplatin-Natriumsalze ist eine andere als die der entsprechenden Base; die Absorption und die Auflösung sowie die Pharmakogenetik der cis-Oxoplatin-Salze ist nicht mit denen der Base vergleichbar. Die Art der Wechselwirkung mit der DNA wie auch die Effizienz und die Wirksamkeit sowie auch die therapeutische Potenz der cis-Oxoplatin-Salze ist eine andere als die von cis-Oxoplatin. Dies kann unter anderem an der chemischen Struktur von cis-Oxoplatin-Calziumsalzen als ein Beispiel von Salzen aus zweiwertigen Kationen gezeigt werden:

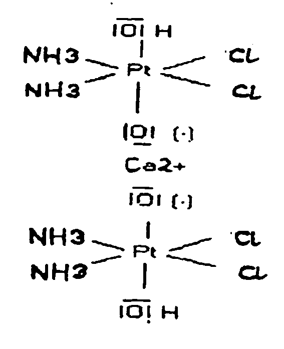

An dieser Struktur zeigt sich, dass Salze wie zum Beispiel Calziumsalze eine vollkommen andere Struktur als die entsprechende Base haben. Diese unterschiedlichen stereochemischen Eigenschaften bewirken ein anderes Verhalten bei der Wechselwirkung mit der DNA in Zellen, insbesondere Krebszellen. Durch die unterschiedliche Struktur der Salze kann eine geringere Dosis ausreichend sein, um einen therapeutischen Effekt zu erzielen. Weiterhin kann die Biotransformation dazu führen, dass die Platin(IV)-Komplexe im Körper zu Platin(II)-Komplexen umgebildet werden, wobei die Platin(IV)- und die Platin(II)-Komplexe auf unterschiedliche Tumoren unterschiedlich wirken (siehe Tabelle I).

### 2. Zytotoxische Aktivität von trans-Oxoplatin(TRAXO)

Trans-Oxoplatin wurde mit einer Anfangskonzentration von 40 g/ml und 2-fachen Schritten gegen Panel f Zelllinien getestet. Da die IC50-Werte in den meisten Fällen nicht erreicht wurden, ist das Überleben der Zellen bei der höchsten Konzentration angegeben.

**Tabelle 3**

| | **Zelllinie** | **% Überleben bei 40 µg/ml TRAXO (außer wenn 20 µm/ml angezeigt sind)** |
|---|---|---|
| U-87-MG | Astrocytoma | 100 |
| ASTRO | Astrocytoma | 82(20)/71 |
| SW620 | Colon carcinoma | 43/51/97 |
| MDA-MB-231 | Brust cancer | 70/103/106 |
| G-292 | Osteosarcoma | 8.6/68 |
| PANC1 | Pancreatic cancer | 100 |
| CRO1A | Carcinoid | 87/104/70 |
| CRO2B | Carcinoid | 24/57 |
| MIAPaCa2 | Pancreatic Cancer | 92/83 |
| Fib3 | Fibroblasts | 91 |
| K562 | Leukaemia | 97 |
| WI-38 | Embry, lung fibroblasts | 21 |
| COLO 205 | Colon carcinoma | 109 |
| HCT-15 | Colon carcinoma | 100 |
| T-47D | Brust cancer | 101 |
| HL-60 | Leukaemia | 0.5 |
| HOS | Osteosarcoma | 4.3 |
| ACHN | Renal carcinoma | 48 |
| BxPC3 | Pancreatic carcinoma | 106 |

Wie durch die Versuche an 19 Zelllinien gezeigt wird, hat TRAXO eine erhebliche Aktivität gegen eine Colon Karzinomzelllinie (SW 620), gegen 2 Osteosarcom-Zelllinien (G-292, HOS), gegen eine Nierenkarzinom-Zelllinie (ACHN), eine Leukämiezelllinie (HL-60) und eine embryonale Lungenfibroblastenzelllinie (WI-38). Zelllinien, die gegen cis-Oxoplatin sensitiv sind, wie beispielsweise T-47D und BxPC3, sind nicht sensitiv gegenüber TRAXO. Die Salze der trans-Oxoplatinverbindungen können ein unterschiedliches therapeutisches Potential und eine andere Wirksamkeit gegen einzelne humane Krebszellen und Zelllinien sowie Tumoren haben.

## Patentansprüche

1. Verfahren zur Herstellung von trans- oder cis-Diammoniumdichlorodihydroxoplatin (IV) und deren Derivaten,
**dadurch gekennzeichnet, dass**
trans- oder cis-Diammoniumdichlorodihydroxoplatin (II) mit einer > 30%iger Peroxid-umfassenden Lösung bei Temperaturen unter 30 °C umgesetzt, das hierdurch gewonnene Produkt mit einer Mineralsäure gelöst und anschließend mit einer alkalischen Lösung ausgefällt wird.

2. Verbindung der allgemeinen Formel I 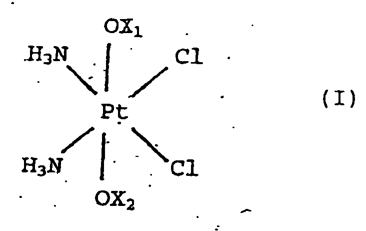 wobei X₁; X₂ ein Kation der 1. bis 5. Hauptgruppe, Alkyl- und/oder Arylreste sein können.

3. Verbindung nach Anspruch 2,
**dadurch gekennzeichnet, dass**
die Kationen Calcium-, Magnesium-, Natrium-, Kalium-, Lithium-Ionen sein können.

4. Verwendung der Verbindung gemäß einem der Ansprüche 2 oder 3 zur Herstellung eines Medikamentes zur Prophylaxe, Therapie, Verlaufskontrolle und/oder Nachbehandlung von mit Zellwachstum, -differenzierung und/oder -teilung im Zusammenhang stehenden Krankheiten.

5. Verwendung nach dem vorhergehenden Anspruch,
**dadurch gekennzeichnet, dass**
die Krankheit ein Tumor ist.

6. Verwendung nach dem vorhergehenden Anspruch,
**dadurch gekennzeichnet, dass**
die Verbindung oral, vaginal, rektal, nasal, subkutan, intravenös, intramuskulär, intraperitoneal, regional und/oder topisch eingesetzt wird.

7. Pharmazeutisches Mittel umfassend eine Verbindung gemäß Anspruch 2 oder 3 gegebenenfalls zusammen mit einem pharmazeutisch verträglichen Träger, Adjuvans und/oder Vehikel.

8. Pharmazeutisches Mittel nach dem vorhergehenden Anspruch,
**dadurch gekennzeichnet, dass**
die Träger ausgewählt sind aus der Gruppe umfassend Füllmittel, Streckmittel, Bindemittel, Feuchthaltemittel, Sprengmittel, Lösungsverzögerer, Resorptionsbeschleuniger, Netzmittel, Adsorptionsmittel und/oder Gleitmittel.

9. Pharmazeutisches Mittel nach einem der vorhergehenden Ansprüche,
**dadurch gekennzeichnet, dass**
die Träger Liposomen, Siosomen und/oder Niosomen sind.

10. Kit umfassend Verbindungen gemäß Anspruch 2 oder 3 gegebenenfalls zusammen mit einer Information zum Kombinieren der Inhalte des Kits.

11. Kit nach dem vorhergehenden Anspruch zur Prophylaxe oder Therapie von Tumorerkrankungen.

## Claims

1. A method for the preparation of *trans*- or *cis*-diammoniumdichlorodihydroxoplatinum (IV) and derivatives thereof,
**characterized in that**
*trans-* or *cis*-diammoniumdichloroplatinum (II) is reacted with a solution comprising >30% peroxide at temperatures below 30°C, and the product thus obtained is dissolved in a mineral acid and subsequently precipitated with an alkaline solution.

2. A compound of general formula I 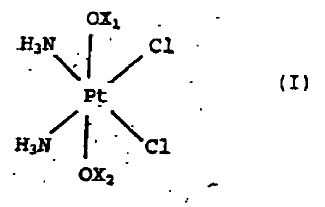 wherein X₁, X₂ can be a cation from the main groups I to V, alkyl and/or aryl residues.

3. The compound according to claim 2,
**characterized in that**
the cations can be calcium, magnesium, sodium, potassium, lithium ions.

4. Use of the compound according to any of claims 2 or 3 in the production of a medicament for prophylaxis, therapy, follow-up and/or aftercare of diseases associated with cell growth, cell differentiation and/or cell division.

5. The use according to the preceding claim,
**characterized in that**
the disease is a tumor.

6. The use according to the preceding claim,
**characterized in that**
the compound is employed orally, vaginally, rectally, nasally, subcutaneously, intravenously, intramuscularly, intraperitoneally, regionally and/or topically.

7. A pharmaceutical agent, comprising a compound according to claim 2 or 3, optionally together with a pharmaceutically tolerable carrier, adjuvant and/or vehicle.

8. The pharmaceutical agent according to the preceding claim,
**characterized in that**
the carriers are selected from the group comprising fillers, diluents, binders, humectants, disintegrants, dissolution retarders, absorption enhancers, wetting agents, adsorbents and/or lubricants.

9. The pharmaceutical agent according to any of the preceding claims,
**characterized in that**
the carriers are liposomes, siosomes and/or niosomes.

10. A kit, comprising the compounds according to claim 2 or 3, optionally together with information for combining the contents of the kit.

11. The kit according to the preceding claim for the prophylaxis or therapy of tumor diseases.

## Revendications

1. Procédé de préparation de trans- ou cis-diammoniumdichlorodihydroxoplatine (IV) et de leurs dérivés,
**caractérisé en ce**
**qu'**on fait réagir du trans- ou cis-diammoniumdichlorodihydroxoplatine (II) avec une solution de peroxyde > 30 % à des températures inférieures à 30°C, on dissous le produit obtenu avec un acide minéral et on le précipite ensuite avec une solution alcaline.

2. Composé de formule générale I 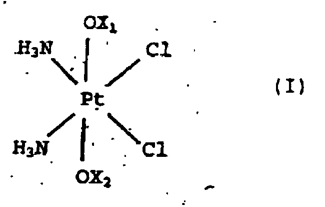 dans laquelle X₁, X₂ peuvent être un cation du groupe principal 1 à 5, des groupements alkyl et/ou aryl.

3. Composé selon la revendication 2,
**caractérisé en ce**
**que** les cations peuvent être des ions calcium, magnésium, sodium, potassium, lithium.

4. Utilisation du composé selon l'une des revendications 2 ou 3 pour préparer un médicament destiné à la prophylaxie, la thérapie, le contrôle de suivi et/ou le traitement consécutif des maladies associées à la croissance cellulaire, la différenciation cellulaire, la division cellulaire.

5. Utilisation selon la revendication précédente,
**caractérisée en ce que**
la maladie est une tumeur.

6. Utilisation selon la revendication précédente,
**caractérisée en ce que**
le composé est appliqué par voie orale, vaginale, rectale, nasale, sous-cutanée, intraveineuse, intramusculaire, intrapéritonéale, régionale et/ou topique.

7. Agent pharmaceutique comprenant un composé selon la revendication 2 ou 3, éventuellement en association avec un excipient, un adjuvant et/ou un vecteur pharmaceutiquement compatible.

8. Agent pharmaceutique selon la revendication précédente,
**caractérisé en ce que**
les excipients sont choisis parmi le groupe comportant des agents de remplissage, des agents délayants, des agents liants, des agents humectants, des agents d'aspersion, des retardateurs de dissolution, des accélérateurs de résorption, des agents mouillants, des agents d'adsorption et/ou des agents lubrifiants.

9. Agent pharmaceutique selon l'une des revendications précédentes,
**caractérisé en ce que**
les vecteurs sont des liposomes, des siosomes et/ou des niosomes.

10. Kit comprenant des composés selon la revendication 2 ou 3 et contenant éventuellement un information pour associer les contenus du kit.

11. Kit selon la revendication précédente pour la prophylaxie ou la thérapie des maladies tumorales.
